# EUROPÄISCHE PATENTSCHRIFT

(11) **EP 3 218 500 B1**
(45) Veröffentlichungstag und Bekanntmachung des Hinweises auf die Patenterteilung: **07.07.2021**
(21) Anmeldenummer: 15831125.8
(22) Anmeldetag: 05.11.2015
(51) Int. Cl.: C12N 15/82, A01N 31/06

(54) **PFLANZEN MIT ERHÖHTER RESISTENZ GEGEN PFLANZENPATHOGENE SOWIE VERFAHREN ZUR ERZEUGUNG ERHÖHTER PATHOGENRESISTENZ IN PFLANZEN**
PLANTS WITH INCREASED RESISTANCE AGAINST PLANT PATHOGENS AND METHODS FOR INCREASING PATHOGEN RESISTANCE IN PLANTS
PLANTES AVEC UNE RÉSISTANCE AUGMENTÉE CONTRE DES AGENTS PATHOGÈNES DE PLANTES ET PROCÉDÉS POUR AUGMENTER LA RÉSISTANCE DE PLANTES CONTRE DES AGENTS PATHOGÈNES DE PLANTES

(30) Priorität: 12.11.2014 DE 102014016774
(43) Veröffentlichungstag der Anmeldung: 20.09.2017
(73) Patentinhaber: Eberhard Karls Universität Tübingen, 72074 Tübingen (DE)
(72) Erfinder: SCHAAF, Gabriel, 72076 Tübingen (DE); LAHA, Debabrata, 53121 Bonn (DE); FREYER, Marc, 72135 Dettenhausen (DE); DE CAMPOS, Marília, K.,F., 71034 Böblingen (DE); JOHNEN, Philipp, 72072 Tübingen (DE); VAN WEES, Saskia, C., M., 3602 PW Maarssen (NL)
(74) Vertreter: Kuttenkeuler, David
(86) Internationale Anmeldenummer: PCT/DE2015/000534
(87) Internationale Veröffentlichungsnummer: WO 2016/074659

(56) Entgegenhaltungen:
- DE-C1- 4 423 022
- MINTU DESAI ET AL: "Two inositol hexakisphosphate kinases drive inositol pyrophosphate synthesis in plants", THE PLANT JOURNAL, Bd. 80, Nr. 4, 17. September 2014 (2014-09-17), Seiten 642-653, XP055257711, GB ISSN: 0960-7412, DOI: 10.1111/tpj.12669
- M. D. CURTIS: "A Gateway Cloning Vector Set for High-Throughput Functional Analysis of Genes in Planta", PLANT PHYSIOLOGY., Bd. 133, Nr. 2, 11. September 2003 (2003-09-11), Seiten 462-469, XP055257706, US ISSN: 0032-0889, DOI: 10.1104/pp.103.027979
- LAURA B. SHEARD ET AL: "Jasmonate perception by inositol-phosphate-potentiated COI1-JAZ co-receptor", NATURE, Bd. 468, Nr. 7322, 6. Oktober 2010 (2010-10-06), Seiten 400-405, XP055257727, United Kingdom ISSN: 0028-0836, DOI: 10.1038/nature09430
- DAKIN K; LI WH: "Cell membrane permeable esters of D-myo-inositol 1,4,5-trisphosphate", CELL CALCIUM, Bd. 42, Nr. 3, 2007, Seiten 291-301, XP022155388, in der Anmeldung erwähnt
- DEBABRATA LAHA ET AL: "VIH2 Regulates the Synthesis of Inositol Pyrophosphate InsP 8 and Jasmonate-Dependent Defenses in Arabidopsis", THE PLANT CELL, Bd. 27, Nr. 4, 21. April 2015 (2015-04-21) , Seiten 1082-1097, XP055257730, US ISSN: 1040-4651, DOI: 10.1105/tpc.114.135160
- MURPHY ALEX M ET AL: "A role for inositol hexakisphosphate in the maintenance of basal resistance to plant pathogens", THE PLANT JOURNAL, BLACKWELL SCIENTIFIC PUBLICATIONS, OXFORD, GB, vol. 56, no. 4 1 November 2008 (2008-11-01), pages 638-652, XP002557384, ISSN: 0960-7412, DOI: 10.1111/J.1365-313X.2008.03629.X [retrieved on 2008-08-20]

## Beschreibung

Die vorliegende Erfindung betrifft Pflanzen mit erhöhter Resistenz gegen herbivore Insekten und/oder pathogene Pilze gemäß den beiliegenden Patentansprüchen.

Pflanzen sind ständig Pflanzenpathogenen ausgesetzt. Insbesondere sind hierbei herbivore Insekten und deren Larven, sowie Pilze und Pilz-ähnliche Erreger (z.B. Oomyceten) zu nennen. Konservative Schätzungen gehen davon aus, dass die agronomischen Schäden, die allein durch Arten der Pilzgattung *Botrytis* verursacht werden, weltweit ca. 1 Milliarde Euro /Jahr betragen¹. Die hierbei entstehenden Schäden werden nicht nur durch direkten Ernteverlust, sondern auch durch den Qualitätsverlust von Produkten (z.B. durch Anreicherung von Mycotoxinen) verursacht.

Eine konventionelle Methode zur Bekämpfung von Insekten und pilzlichen Erregern ist die Verwendung von chemischen Pflanzenschutzmitteln, wobei unter diesen Maßnahmen häufig ein Teil des Ertrags verloren geht. Der Einsatz chemischer Pflanzenschutzmittel bei Nutzpflanzen kann negative Auswirkungen auf Menschen und Umwelt haben. Außerdem sind diese Maßnahmen sehr kosten- und arbeitsintensiv. Darüber hinaus entwickeln die mit chemischen Pflanzenschutzmitteln zu bekämpfenden Krankheitserreger häufig Anpassungsmechanismen, so dass solche Maßnahmen oft nicht die gewünschte Schutzwirkung erzielen.

Eine Alternative zum Einsatz chemischer Mittel ist die Nutzung Insekten- und Pilzresistenter Sorten. Eine Züchtung neuer Sorten mittels der herkömmlichen Pflanzenzüchtung ist wegen der Komplexität von Pflanzengenomen sehr langwierig und schwierig. Die herkömmliche Pflanzenzüchtung nutzt meist spontane oder induzierte Mutationen, deren Ausprägungen ungezielt durch äußere Einflüsse (z. B. Kälteschocks oder radioaktive Bestrahlung) hervorgerufen werden.

Eine Alternative zur herkömmlichen Züchtung stellt die Herstellung gentechnisch veränderter Pflanzen dar. Dabei werden in das Erbgut der Pflanzen gezielt Gene mit gewünschten Eigenschaften eingeschleust. Derzeit werden als gentechnisch veränderte Pflanzen insbesondere herbizid- und insektenresistente Pflanzensorten vermarktet. Das Ziel der neuen Sortenherstellung ist dabei die Ertragssteigerung unter widrigen Bedingungen, wie unter Trockenstress oder Insektenbefall.

Vor allem werden derzeit gentechnisch veränderte Mais- und Baumwollpflanzen mit Insektenresistenz angebaut. In den meisten Fällen leitet sich diese Eigenschaft aus dem Bt-Toxin ab, welches von einem in die Pflanzen übertragenen Gen kodiert wird. Das Gen stammt aus dem Bodenbakterium *Bacillus thuringiensis,* welches diesen Wirkstoff natürlicherweise produziert. Eines der Probleme dieser Strategie ist jedoch, dass sich bereits die ersten Resistenzen bei Pflanzenschädlingen entwickelt haben.

Außerdem wurde in vielen wissenschaftlichen Arbeiten gezeigt, dass der Bt-Mais Schmetterlinge schädigt und zahlreiche andere Nichtzielorganismen gefährdet. Bt-Toxine werden nur langsam abgebaut, reichern sich im Boden an und werden in der Nahrungskette weitergegeben. Ähnliche wie der Einsatz von Bt-Toxinen im biologischen Pflanzenschutz, sowie bei der Bekämpfung von Stechmücken, birgt daher auch der Anbau von Bt-Mais demzufolge vielfältige Risiken für die Artenvielfalt.

Murphy Alex M et al., The Plant Journal, Bd. 56, Nr. 4, 2008-11-01, Seiten 638-652, offenbaren eine Assoziation der Pflanzenabwehr und InsP₆.

Mintu Desai et al., The Plant Journal 2014, Bd 80, Seiten 642-653, offenbaren die gene AtVip1 und AtVip2 als mögliche InsP₆ Kinasen in Pflanzen.

Die Aufgabe der vorliegenden Erfindung ist es daher, Pflanzen mit erhöhter Resistenz gegen Pflanzenpathogene, sowie Verfahren zur deren Herstellung bereit zu stellen, denen die Nachteile der aus dem Stand der Technik bekannten längerfristigen Toxizität von Wirkstoffen, z.B. durch Anreicherung im Boden, nicht anhaften. Darüber hinaus soll durch Bereitstellen solcher Pflanzen bzw. Verfahren das Spektrum möglicher Maßnahmen gegen Pathogene erweitert und dadurch die Wahrscheinlichkeit der Resistenzentwicklung minimiert werden.

Diese Aufgabe wird durch die Bereitstellung der Pflanzen mit erhöhter Resistenz gegen herbivore Insekten und/oder pathogene Pilze, bei der die intrazelluläre Konzentration von Inositolpyrophosphaten InsP₇ und / oder InsP₈ im Vergleich zur Wildtyppflanze erhöht ist gelöst, wobei die Pflanze durch heterologe Expression der Nukleotid-Sequenz nach der GenBank Accession: At3g01310 gekennzeichnet ist, und dadurch gekennzeichnet ist, dass die Expression eines durch die Nukleotid-Sequenz nach der GenBank Accession: At3g01310 kodierten Proteins VIH2, oder eines homologen Proteins, welches in der Lage ist, Inositolpyrophosphate InsP₇ und / oder InsP₈, zu synthetisieren, in der gesamten Pflanze oder in spezifischen Geweben induzierbar oder im Vergleich zur Wildtyppflanze erhöht ist, wobei die Nukleotid-Sequenz einem anderen Organismus entstammt.

In tierischen Systemen wurden Inositolpyrophosphate als wichtige intrazelluläre Signalmoleküle beschrieben. In der vorliegenden Erfindung wurde zum ersten Mal gezeigt, dass die Proteine VIH2 und VIH1 die Phosphorylierung von Inositolpyrophosphaten InsP₆ und InsP₇ katalysieren können und dass VIH2 in *Arabidopsis-Sämlingen* vor allem für die Synthese des Inositolpyrophosphats InsP₈ verantwortlich ist. Das *VIH2*-Transkript wird in erster Linie in verschiedenen vegetativen Geweben exprimiert und sowohl durch mechanische Verwundung, als auch durch Raupenbefall induziert, wohingegen *VIH1*-Transkript vor allem in den Pollen akkumuliert.

In der vorliegenden Erfindung konnte gezeigt werden, dass VIH2 (GenBank Accession: At3g01310) an der Pathogenabwehr in Pflanzen beteiligt ist (s. Ausführungsbeispiele).

Die Nukleotid-Sequenz stammt aus einem anderen Organismus, wird also heterolog exprimiert. Die heterologe Expression kann hierbei von Vorteil sein, da postranskriptionelle bzw. postranslationale Regulationsmöglichkeiten im Wirtsorganismus (z.B. Deaktivierung des Enzyms bei Überproduktion) häufig umgangen werden können.

Die Induzierbarkeit von VIH2, oder eines homologen Proteins, kann durch die dem Fachmann bekannten Verfahren erreicht werden. So kann die Expression entsprechender Nukleotid-Sequenzen beispielsweise unter der Kontrolle eines induzierbaren Promotors in der Zielpflanze erfolgen. Bekannte induzierbare Expressionssysteme, die bereits erfolgreich in *Arabidopsis,* Tabak, Reis, oder Mais verwendet wurde, bestehen normalerweise aus zwei Komponenten: einem (häufig chimären) Transkriptionsfaktor, der konstitutiv oder gewebespezifisch exprimiert wird, und dem eigentlichen Promotor, der die Expression der erwünschten Nukleotid-sequenz reguliert. Dieser Promoter kann vom chimären Transkriptionsfaktor durch einen externen Stimulus aktiviert werden. Bekannte Beispiele sind Ethanolinuzierbare ("AlcR/AlcA"-System), Dexamethasone-induzierbare (GR-Fusionen, GVG- und pOp/LhGR-Systeme), β-Estradiol-induzierbare (XVE/OlexA-System) und Hitzeschock-induzierbare Expressionssysteme.

Zur induzierten Expression könnten auch in der Zielpflanze natürlich vorkommende Promotoren, die beispielsweise durch Pathogene induziert werden, verwendet werden. Bekannte Beispiele sind z.B. Promotoren, welche die Expression der Transkripte der im Jasmonat-Haushalt wichtigen JAZ ("jasmonate ZIM-domain")-Proteine regulieren und in allen höheren Pflanzen vorkommen.

Die erhöhte Expression (Überexpression) von VIH2, oder eines homologen Proteins, kann durch die dem Fachmann bekannten Verfahren erreicht werden. So kann die Expression entsprechender Nukleotid-Sequenzen unter der transkriptionellen Kontrolle eines konstitutiven Promotors erfolgen, z.B. des Blumenkohlmosaikvirus-Promotors CaMV 35S oder eines Ubiquitin (UBQ)-Promotors. Denkbar ist auch die Verwendung gewebespezifischer Promotoren, die z.B. nur in den potentiell durch Pathogen zu befallenen Geweben natürlicherweise von der Pflanze exprimiert werden, wie beispielsweise blatt-, frucht- oder samenspezifische Promotoren.

Bei den Pflanzenpathogenen, gegen die die erfindungsgemäße Pflanze resistent ist, handelt es sich um herbivore Insekten, z.B. Larven von landwirtschaftlich relevanten Schädlingen, wie des kleinen Kohlweißlings oder des Eulenfalters, sowie um pathogene Pilze, wie nekrotrophe Pilze, z.B. Vertreter der Gattungen *Alternaria* oder *Botrytis.*

Der Vorteil der vorliegenden Erfindung gegenüber den aus dem Stand der Technik bekannten Verfahren, wie z.B. der Expression des Bt-Toxins aus dem Bakterium *Bacillus thuringiensis* in Pflanzen, besteht darin, dass in der erfindungsgemäßen Pflanze endogene Mechanismen zur Erhöhung von Resistenz gegen Pflanzenpathogene und Umweltstress vorteilhaft ausgenutzt werden können. Weil es dabei um pflanzeneigene und nicht um artfremde Gene und Substanzen geht, kann davon ausgegangen werden, dass die Akzeptanz solcher Pflanzen in der Bevölkerung besser ist, als es bei herkömmlichen genetisch veränderten Pflanzen der Fall ist. Außerdem ist nicht zu erwarten, dass der landwirtschaftliche Einsatz erfindungsgemäßer Pflanzen gravierende Auswirkungen auf Nichtzielorganismen und auf die Umwelt haben wird, wie die Nutzung herkömmlicher Insektizide oder der Anbau von Bt-Pflanzen. Ferner kann davon ausgegangen werden, dass die Resistenzbildung von Schädlingen im Gegensatz zu herkömmlichen Verfahren zur Erzeugung resistenter Pflanzen langsamer auftritt, da die gesamte pflanzliche Abwehrmaschinerie gegenüber Pathogenen durch Inositolpyrophosphate induziert werden kann und nicht nur einzelne Toxine.

Weitere Vorteile, Merkmale und Anwendungsmöglichkeiten der Erfindung werden nachstehend anhand des unten beschriebenen Ausführungsbeispiels mit Bezug auf die Figuren beschrieben.

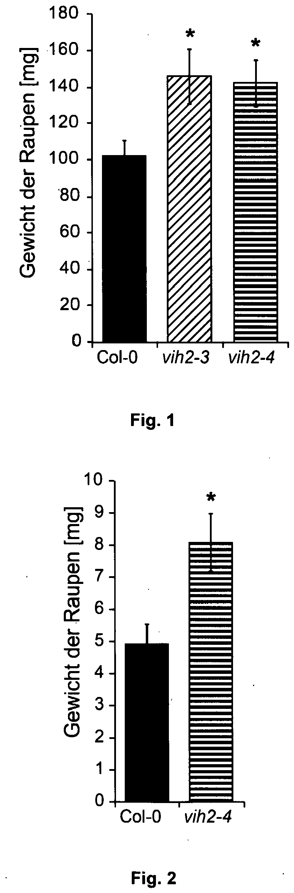

### Fig. 1: Arabidopsis VIH2 Funktionsverlustmutanten zeigen eine verminderte Resistenz gegen Larven des Kleinen Kohlweißlings (Pieris rapae). Die

Larvenentwicklung wurde in einem sogenannten 'no choice' Assay untersucht. Hierbei wird je eine Raupe im Larvenstadium L1 auf eine einzelne, 5-Wochen alte Pflanze des angegebenen Genotyps gebracht, und durch eine Gaze am Entkommen gehindert. Col-0 bezeichnet den verwendeten Wildtyp *Arabidopsis thaliana* (Acker-Schmalwand) Ökotyp, *vih2-3* und *vih2-4* sind VIH2 Funktionsverlustmutanten im Col-0 Hintergrund. Das Frischgewicht der Raupen wurde nach 7 Tagen bestimmt. Die Werte geben den Mittelwert (± Standardfehler) an (n=20). Statistisch signifikante Unterschiede werden durch ein Sternchen angezeigt (t-Test; * p < 0.02).

### Fig. 2: Arabidopsis VIH2 Funktionsverlustmutante zeigt eine verminderte Resistenz gegen Larven des Eulenfalters Mamestra brassicae (Kohleule). Die

Larvenentwicklung wurde in einem sogenannten 'no choice' Assay untersucht. Hierbei wird je eine Raupe im Larvenstadium L1 auf eine einzelne, 5-Wochen alte Pflanze des angegebenen Genotyps gebracht, und durch eine Gaze am Entkommen gehindert. Col-0 bezeichnet den verwendeten Wildtyp *Arabidopsis thaliana* (Acker-Schmalwand) Ökotyp, *vih2-4* ist eine VIH2 Funktionsverlustmutanten im Col-0 Hintergrund. Das Frischgewicht der Raupen wurde nach 8 Tagen bestimmt. Die Werte geben den Mittelwert (± Standardfehler) an (n=20). Statistisch signifikante Unterschiede werden durch ein Sternchen angezeigt (t-Test; * p < 0.02).

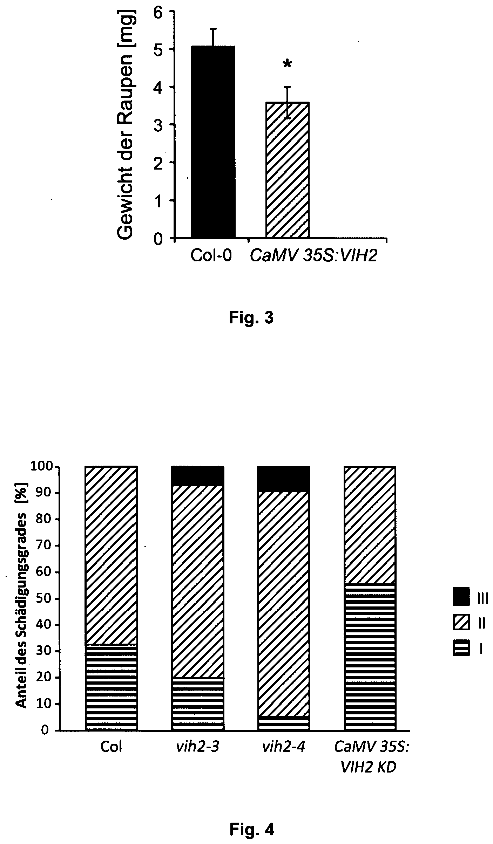

### Fig. 3: Arabidopsis VIH2 überexprimierende Linien zeigen eine erhöhte Resistenz gegen Larven des Eulenfalters Mamestra brassicae (Kohleule). Die

Larvenentwicklung wurde in einem sogenannten 'no choice' Assay untersucht. Hierbei wird je eine Raupe im Larvenstadium L1 auf eine einzelne, 5-Wochen alte Pflanze des angegebenen Genotyps gebracht, und durch eine Gaze am Entkommen gehindert. Col-0 bezeichnet den verwendeten Wildtyp *Arabidopsis thaliana* (Acker-Schmalwand) Ökotyp, *"CaMV 35S: VIH2"* sind transgene Pflanzen, in denen die Kinasedomäne des wildtypischen *VIH2* Gens unter der Kontrolle des starken, viralen *CaMV 35S* Promotors überexprimiert wird. Das Frischgewicht der Raupen wurde nach 8 Tagen bestimmt. Die Werte geben den Mittelwert (± Standardfehler) an (n=20). Statistisch signifikante Unterschiede werden durch ein Sternchen angezeigt (t-Test; ^{*} p < 0.05). Diese Versuche zeigen, dass eine Erhöhung der Expression von VIH2 (welche mit einer Erhöhung des Inositolpyrophosphats InsP₈ verbunden ist) zu einer erhöhten Resistenz der Pflanzen gegenüber herbivoren Insektenschädlingen führt. Das Wachstum von Schädlingen auf den transgenen Pflanzen (und in Annäherung daran der Schädlings-induzierte Schaden) ist um ca. 30 % verringert. Hierbei gibt es keine "unerwünschten" Nebeneffekte der Inositolpyrophosphat-Erhöhung: Die Pflanzen sind gesund und entwickeln und vermehren sich normal.

### Fig. 4: In Arabidopsis führt Überexpression von VIH2 zur erhöhten Resistenz gegen den nekrotrophen Ascomycet Alternaria brassicicola während VIH2 Funktionsverlustmutanten eine verminderte Resistenz zeigen

Infektionsversuche mit *Alternaria brassicicola* (Isolat MUCL 20297) wurden wie zuvor beschrieben durchgeführt³. Hierzu wurden Sporen auf eine Dichte von 5 × 10⁵ Sporen/mL eingestellt, vier bis sechs 5 µL-Tropfen der Sporensuspension auf die Blattoberfläche aufgebracht und die Pflanzen bei 100 % Luftfeuchte bei 22°C und einem 8-Stunden/16-Stunden Licht/Dunkelrhythmus für 7-10 Tage inkubiert (Anzahl der Pflanzen ≥ 15). Anschließend wurden die Krankheitssymptome dokumentiert und in verschiedene Klassen je Phänotyp der Blattschädigung eingeteilt. Die Klassen waren hierbei wie folgt. Klasse I (quer gestreift): Hellbraune Flecken an der Infektionsstelle; Klasse II (schräg gestreift):Dunkelbraune Flecken an der Infektionsstelle und erste Anzeichen von Nekrose; Klasse III (durchgehend schwarz): fortschreitende Nekrose und Blattmazeration. Die Verteilungen der Daten wurde mit einem Chi-Quadrat-Test ausgewertet und zeigen, dass die Unterschiede zwischen Col-0 und *vih2-3,* zwischen Col-0 und *vih2-4,* sowie zwischen Col-0 und *CaMV 35S:VIH2* signifikant sind (p < 0.05). Bei *vih2-3* und *vih2-4* handelt es sich um VIH2 Funktionsverlustmutanten, bei *CAMV 35S: VIH2* handelt es sich um transgene Pflanzen, in denen die Kinasedomäne des wildtypischen *VIH2* Gens unter der Kontrolle des starken, viralen *CaMV 35S* Promotors überexprimiert wird. Diese Versuche zeigen, dass eine Erhöhung der Expression von VIH2 (welche mit einer Erhöhung des Inositolpyrophosphats InsP₈ verbunden ist) zu einer erhöhten Resistenz der Pflanzen gegenüber nektrotrophen Pilzen führt, da die Pilz-induzierten Schäden auf solchen Pflanzen signifikant verringert sind.

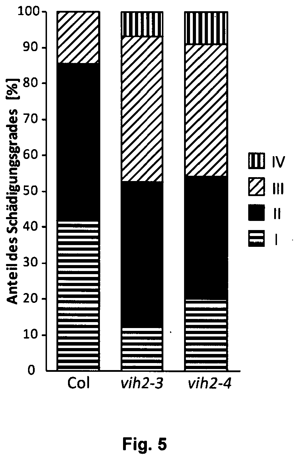

### Fig. 5: Arabidopsis VIH2 Funktionsverlustmutanten zeigen eine verminderte Resistenz gegen den nekrotrophen Pilz und Erreger der Grauschimmelfäule

***Botrytis cinerea.*** Ein 5 µL großer Tropfen einer Konidien (Sporen)-Suspension von *Botrytis cinerea* wurde auf die Blattoberfläche einer 5-Wochen alten Pflanzen pipettiert. Dies wurde für 5 ausgewachsene Blätter pro Pflanze und insgesamt 20 Pflanzen des angezeigten Genotyps durchgeführt. Hierbei wurden nur Blätter verwendet, die jünger sind als das 4. Blatt der jeweiligen Pflanze. Danach wurden die inokulierten Pflanzen bei 100 % Luftfeuchte für 3 Tage bei 21°C in einer Klimakammer unter einem 10-Stunden/14-Stunden Licht/Dunkelrhythmus inkubiert. Anschließend wurden die Krankheitssymptome dokumentiert und in verschiedene Klassen je nach Größe und dem Zeitpunkt des Auftretens von Läsionen eingeteilt.

Die Klassen waren hierbei wie folgt: Klasse I (quer gestreift): Läsionen von 2 mm Durchmesser; Klasse II (durchgehend schwarz): Läsionen von 2 mm Durchmesser mit Chlorose; Klasse III (schräg gestreift): Läsionen von 2-4 mm Durchmesser mit Chlorose; Klasse IV (längs gestreift): Läsionen mit einem Durchmesser > 4 mm und Chlorose. Die Verteilungen der Daten wurde mit einem Chi-Quadrat-Test ausgewertet und zeigen, dass die Unterschiede zwischen Col-0 und *vih2-3* sowie zwischen Col-0 und *vih2-4* signifikant sind (p < 0.001). Die Konidiensuspensionen für diese Versuche wurden, wie zuvor beschrieben⁴, hergestellt. Hierzu wurden Konidien von *Botrytis cinerea* aus einem Glycerolstock auf halb-konzentriertes 'potato dextrose broth' Festmedium (PDB; Difco TM) inokuliert und 2 Wochen bei 22°C unter einem 10-Stunden/14-Stunden Licht/Dunkelrhythmus inkubiert. Danach wurden Konidien von der Oberfläche des Festmediums mit halb-konzentriertem PDB-Flüssigmedium gewaschen, durch Glaswolle gefiltert und die Konidiendichte in einer Zählkammer bestimmt. Die Suspension wurde in halb-konzentriertem PDB-Flüssigmedium auf eine Dichte von 5 × 10⁵ Konidien/mL eingestellt, 2 Stunden bei Raumtemperatur inkubiert und zur Blattinokulation verwendet. Die Versuche wurden mit ähnlichen Ergebnissen 3-mal wiederholt.

### Ausführunqsbeispiele

Die Versuche wurden mit isogenen Linien des gleichen Ökotyps (*Arabiopsis thaliana,* Col-0) durchgeführt, die sich durch Anwesenheit (Col-0) bzw. Abwesenheit (*vih2-3* und *vih2-4*) eines intakten *VIH2* Gens (und damit VIH2-Proteins), oder aber durch eine erhöhte Expression der *VIH2* Kinasedomäne (*CaMV 35S: VIH2*) auszeichnen. Bei den letztgenannten Pflanzen (*CaMV 35S: VIH2*) wurde die Kinasedomäne des wildtypischen *VIH2* Gens unter der Kontrolle des starken, viralen *CaMV 35S* Promotors überexprimiert. Hierzu wurde die die *VIH2* Kinasedomänesequenz von einer Arabidopsis cDNA amplifiziert und in den Vektor pENTR™/D-TOPO® (Invitrogen Life Technologies) integriert. Von dort wurde die *VIH2* Kinasedomänesequenz durch Gateway® LR Clonase™ II (Invitrogen Life Technologies) in den binären Pflanzentransformationsvektor pGWB441 (Nakagawa et al., 2007, Biosci. Biotechnol. Biochem, 71, 2095-2100) überführt und der hierdurch hergestellte Vektor ,pGWB441-VIH2 KD' zur Transformation von Arabidopsispflanzen verwendet. Es wurden mehrere unabhängige Transformanten auf Kanamycin selektioniert, nicht mehr segregierende ***CaMV35S:VIH2*** T3 Pflanzen etabliert und eine erhöhte InsP₈ Biosynthese in diesen Pflanzen bestätigt. In den Figuren 3 und 4 werden exemplarisch Versuche mit einer dieser Linien gezeigt, die demonstrieren, dass die erhöhte Expression der VIH2 Kinasedomäne (und damit einhergehend die erhöhte Produktion von InsP₈) zu einer erhöhten Resistenz sowohl gegen Larven des herbivoren Insekts *Mamestra brassicae* (Kohleule, Fig. 3) als auch gegen den nekrotrophen Pilz *Alternaria brassicicola* (Fig. 4) führt. Die verwendeten VIH2 Funktionsverlustpflanzen (*vih2-3 und vih2-4*) stammen aus öffentlich zugänglichen Samen-Repositorien wie nachführend angegeben: ***Arabidopsis thaliana* Col-0** (Columbia-0, CS60000 dessen Genom vollständig sequenziert ist) vom 'Salk Institute Genomic Analysis Laboratory' (USA); ***vih2-3*** (SAIL_165_F12) von der 'Syngenta Arabidopsis Insertion Library (SAIL)' Sammlung. Diese Linie wurde uns über das 'Arabidopsis Biological Research Center' (ABRC) der 'Ohio State University' (USA) verfügbar gemacht; ***vih2-4*** (GK-080A07) stammt aus der Kollektion ,Genomanalyse im biologischen System Pflanze (GABI-KAT)' der Universität Bielefeld. In beiden Fällen (SAIL und GABI-KAT) wurden *Arabidopsis thaliana* Col-0-Pflanzen durch Transformation mit spezifischen T-DNA-enthaltenden Vektoren mit Hilfe von Agrobakterien transformiert. Die T-DNAs integrieren hierbei weitestgehend ungerichtet ins Genom und können je nach genomischem Lokus zur Zerstörung des betroffenen Gens (und damit zum Verlust des durch dieses Gen kodierten Proteins) führen. Eine Vielzahl solcher Pflanzen werden in den entsprechenden Repositorien mit Hilfe T-DNA-spezifischer Oligonukleotide sequenziert, um die Insertionsstelle und damit die Identität des betroffenen Gens in einer spezifischen Pflanze zu ermitteln. Entsprechende Daten werden online zur Verfügung gestellt, um damit die Identifizierung einer potentiellen Funktionsverlustmutante des gewünschten Proteins zu ermöglichen. Dieser Umweg (ungerichtete Insertion und nachfolgende Genotypisierung) ist notwendig, da in höheren Pflanzen eine gezielte Herstellung von ,knockout'-Pflanzen durch das Prinzip der homologen Rekombination (im Unterschied beispielsweise zur Bäckerhefe oder zu Mäusen) sehr ineffektiv ist.

Die Ausführungsbeispiele weisen darauf hin, dass VIH2-Funktionsverlustmutanten eine verminderte Resistenz gegenüber herbivoren Insekten und nektrotrophen Pilzen besitzen **(****Fig. 1, 2****,** **4** und **5**), während die erhöhte Expression der VIH2 Kinasedomäne (und damit einhergehend die erhöhte Produktion von InsP₈) eine erhöhte Resistenz gegenüber herbivoren Insekten und nektrotrophen Pilzen bewirkt **(****Fig. 3** und **4****).**

### Referenzen

1. Dean R, Van Kan JA, Pretorius ZA, Hammond-Kosack KE, Di Pietro A, Spanu PD, et al. The Top 10 fungal pathogens in molecular plant pathology. Mol Plant Pathol 2012, 13(4): 414-430.
2. Dakin K, Li WH. Cell membrane permeable esters of D-myo-inositol 1,4,5-trisphosphate. Cell calcium 2007, 42(3): 291-301.
3. Kemmerling B, Schwedt A, Rodriguez P, Mazzotta S, Frank M, Abu Qamar S, et al. The BRl1-associated kinase 1, BAK1, has a Brassinoli-independent role in plant cell-death control. Current Biology 2007, 17(13): 1116-1122.
4. Van Wees SC, Van Pelt JA, Bakker PA, Pieterse CM. Bioassays for assessing jasmonate-dependent defenses triggered by pathogens, herbivorous insects, or beneficial rhizobacteria. Methods Mol Biol 2013, 1011: 35-49.

## Patentansprüche

1. Pflanze mit erhöhter Resistenz gegen herbivore Insekten und/oder pathogene Pilze, bei der die intrazelluläre Konzentration von Inositolpyrophosphaten InsP₇ und / oder InsP₈ im Vergleich zur Wildtyppflanze erhöht ist; wobei die Pflanze durch heterologe Expression der Nukleotid-Sequenz nach der GenBank Accession: At3g01310 gekennzeichnet ist, und **dadurch gekennzeichnet ist, dass** die Expression eines durch die Nukleotid-Sequenz nach der GenBank Accession: At3g01310 kodierten Proteins VIH2, oder eines homologen Proteins, welches in der Lage ist, Inositolpyrophosphate InsP₇ und / oder InsP₈, zu synthetisieren, in der gesamten Pflanze oder in spezifischen Geweben induzierbar oder im Vergleich zur Wildtyppflanze erhöht ist, wobei die Nukleotid-Sequenz einem anderen Organismus entstammt.

2. Pflanze nach Anspruch 1, wobei die Nukleotid-Sequenz unter der Kontrolle eines induzierbaren Promotors steht.

3. Pflanze nach einem der Ansprüche 1 oder 2, wobei die Nukleotid-Sequenz unter der transkriptionellen Kontrolle eines konstitutiven Promotors steht.

4. Pflanze nach Anspruch 2, wobei der Promotor gewebespezifisch ist.

5. Pflanze nach Anspruch 3 oder 4, wobei der Promoter blatt-, frucht- oder samenspezifisch ist.

6. Pflanze nach einem der Ansprüche 1 bis 5, wobei die herbivoren Insekten Larven von landwirtschaftlich relevanten Schädlingen sind, ausgewählt aus der Gruppe umfassend den kleinen Kohlweißling und den Eulenfalter; und/oder wobei die pathogene Pilze nekrotrophe Pilze sind, ausgewählt aus der Gruppe umfassend Vertreter der Gattungen Alternaria oder Botrytis.

## Claims

1. Plant having increased resistance to herbivorous insects and/or pathogenic fungi, in which plant the intracellular concentration of inositol pyrophosphates InsP₇ and/or InsP₈ is increased compared to the wild-type plant; the plant being **characterized by** heterologous expression of the nucleotide sequence according to the GenBank Accession: At3g01310, and **characterized in that** the expression of a protein VIH2 encoded by the nucleotide sequence according to the GenBank Accession: At3g01310, or of a homologous protein which is able to synthesize inositol pyrophosphate InsP₇ and/or InsP₈, is inducible in the whole plant or in specific tissues or is increased compared to the wild-type plant, in which the nucleotide sequence originates from another organism.

2. Plant of claim 1, wherein the nucleotide sequence is under the control of an inducible promoter.

3. Plant according to claim 1 or 2, wherein the nucleotide sequence is under the transcriptional control of a constitutive promoter.

4. Plant according to claim 2, wherein the promoter is tissue-specific.

5. Plant according to claim 3 or 4, wherein the promoter is leaf-, fruit- or seed-specific.

6. Plant according to any of claims 1 to 5, wherein the herbivorous insects are larvae of agriculturally relevant pests selected from the group comprising the cabbage white butterfly and the owlet moth; and/or wherein the pathogenic fungi are necrotrophic fungi selected from the group comprising members of the genera *Alternaria* or *Botrytis.*

## Revendications

1. Plante à résistance accrue aux insectes herbivores et/ou aux champignons pathogènes, selon laquelle la concentration intracellulaire d'inositol pyrophosphates InsP₇ et/ou InsP₈ est accrue par rapport à une plante de type sauvage ; la plante étant **caractérisée par** l'expression hétérologue de la séquence nucléotidique selon le numéro d'accession GenBank : At3g01310, et est **caractérisée en ce que** l'expression d'une protéine VIH2 codée par la séquence nucléotidique selon le numéro d'accession GenBank: At3g01310, ou d'une protéine homologue capable de synthétiser de l'inositol pyrophosphate InsP₇ et/ou InsP₈, peut être induite dans la plante entière ou dans des tissus spécifiques ou est accrue par rapport à une plante de type sauvage, selon laquelle la séquence nucléotidique provient d'un autre organisme.

2. Plante selon la revendication 1, dans laquelle la séquence nucléotidique est sous le contrôle d'un promoteur inductible.

3. Plante selon l'une des revendications 1 ou 2, dans laquelle la séquence nucléotidique est sous le contrôle transcriptionnel d'un promoteur constitutif.

4. Plante selon la revendication 2, dans laquelle le promoteur est tissu-spécifique.

5. Plante selon la revendication 3 ou 4, dans laquelle le promoteur est feuille-spécifique, fruit-spécifique ou graine-spécifique.

6. Plante selon l'une quelconque des revendications 1 à 5, dans laquelle les insectes herbivores sont des larves de ravageurs pertinents pour l'agriculture choisies dans le groupe comprenant la petite piéride du chou et le Noctuidae ; et/ou les champignons pathogènes étant des champignons nécrotrophes choisis dans le groupe comprenant des représentants des espèces *Alternaria* ou Botrytis.
